# EUROPEAN PATENT SPECIFICATION

(11) **EP 3 538 118 B1**
(45) Date of publication and mention of the grant of the patent: **03.03.2021**
(21) Application number: 17809182.3
(22) Date of filing: 10.11.2017
(51) Int. Cl.: A61K 35/74, G01N 33/00, A61K 9/00

(54) **SAPROPHYTIC LIVE BACTERIA FOR USE IN TREATMENT OF HIDRADENITIS SUPPURATIVA**
SAPROPHYTEN-LEBENDBAKTERIEN ZUR VERWENDUNG BEI DER BEHANDLUNG VON HIDRADENITIS SUPPURATIVA
BACTÉRIES SAPROPHYTES VIVANTES DESTINÉES À ÊTRE UTILISÉES DANS LE TRAITEMENT DE L'HIDROSADÉNITE SUPPURÉE

(30) Priority: 11.11.2016 DK PA201670894
(43) Date of publication of application: 18.09.2019
(73) Proprietor: Sjaellands Universitetshospital Dermatologisk Afdeling, 4000 Roskilde (DK); Jemec, Gregor Borut Ernst, 2920 Charlottenlund (DK); Ring, Hans Christian, 2820 Gentofte (DK)
(72) Inventor: RING, Hans Christian, 2820 Gentofte (DK); JEMEC, Gregor Borut Ernst, 2920 Charlottenlund (DK)
(74) Representative: Plougmann Vingtoft a/s
(86) International application number: PCT/DK2017/050369
(87) International publication number: WO 2018/086669

(56) References cited:
- WO-A1-2013/153358
- WO-A1-2016/172196
- WO-A2-2015/026235
- H RING ET AL: "Normal Skin Microbiota is Altered in Pre-clinical Hidradenitis Suppurativa", ACTA DERMATO-VENEREOLOGICA., vol. 97, no. 2, 5 July 2016 (2016-07-05), pages 208-213, XP055448286, United Kingdom ISSN: 0001-5555, DOI: 10.2340/00015555-2503
- HANS CHRISTIAN RING ET AL: "The Follicular Skin Microbiome in Patients With Hidradenitis Suppurativa and Healthy Controls", JAMA DERMATOLOGY, vol. 153, no. 9, 1 September 2017 (2017-09-01), page 897, XP055448288, US ISSN: 2168-6068, DOI: 10.1001/jamadermatol.2017.0904
- Www Revmed Ch: "REVUEMÉDICALESUISSE", , 30 March 2016 (2016-03-30), XP55450330, Retrieved from the Internet: URL:https://www.researchgate.net/profile/J eremy_Di_Domizio/publication/301657909_The _skin_microbiota_A_colossus_steps_into_the _spotlight/links/57e514f708aed68d52f2ab6f/ The-skin-microbiota-A-colossus-steps-into- the-spotlight.pdf?_sg 0!=HufwLPdkl5UUPSWMWaWEXK8kwPBKqtOBraExw8V 0u_VFGIK1vqvh3W5nCVvyxKRY9Fe_o [retrieved on 2018-02-12]
- WILLIAMS MICHAEL R ET AL: "The Role of the Skin Microbiome in Atopic Dermatitis", CURRENT ALLERGY AND ASTHMA REPORTS, CURRENT SCIENCE, US, vol. 15, no. 11, 24 September 2015 (2015-09-24), pages 1-10, XP035602255, ISSN: 1529-7322, DOI: 10.1007/S11882-015-0567-4 [retrieved on 2015-09-24]
- Anonymous: "Are skincare products with probiotics worth the hype? | Dermatology Times", , 8 August 2016 (2016-08-08), XP055450318, Retrieved from the Internet: URL:http://dermatologytimes.modernmedicine .com/dermatology-times/news/skincare-produ cts-probiotics [retrieved on 2018-02-12]
- R. ANDERSEN ET AL: "New treatment strategies for hidradenitis suppurativa", DRUGS OF TODAY, vol. 52, no. 8, 1 January 2016 (2016-01-01), page 439, XP055448284, ES ISSN: 1699-3993, DOI: 10.1358/dot.2016.52.8.2517967
- Riverain-Gillet E et al.: Skin dysbiosis in Hidradenitis Suppurativa:a prospective metagenomic study , April 2017 (2017-04), XP002778137, Retrieved from the Internet: URL:https://www.escmid.org/escmid_publicat ions/escmid_elibrary/material/?mid=52478 [retrieved on 2018-02-12]

## Description

### Technical field of the invention

The present invention relates to specifically defined saprophytic live bacteria originating from healthy human donors for use in topical treatment of Hidradenitis Suppurativa

### Background of the invention

Hidradenitis Suppurativa (HS) is a chronic, inflammatory, skin disease of the hair follicle defined by recurrent nodules, tunnels (sinus tracts) and scarring involving the intertriginous regions. The nodules may progressively expand to abscesses and subsequently rupture, causing suppuration and malodours discharge. The prevalence of HS has been estimated in 1-4% of the European populations (Jemec, 2012).

Although the clinical picture of HS lesions appears reminiscent of bacterial infection, the structure of the bacterial population as well as the diversity at strain level is poorly investigated in HS patients. Previous studies have been restricted to lesional skin without inclusion of healthy control groups, and microbiological investigations of clinically normal HS skin of the intertriginous regions are limited (Ring *et al.*, 2015).

The role of the cutaneous commensal bacterial community (microbiota) and its potential relationship to the innate immune system in HS seems to be of increasing interest. Several studies have hypothesized that a synergistic interaction between the commensal microbiota and aberrant innate immune responses could be a pivotal factor in the pathogenesis of HS. Moreover, increasing evidence imply that aberrant immune responses play a role involving both the innate and adaptive immune system. In particular, recent studies showing efficacy of immunomodulatory agents such as oral corticosteroids, ciclosporin, and lately anti-tumor necrosis factor therapy, further support the hypothesis of a dysfunctional immune system in HS patients.

The majority of previous studies on the bacteriology in HS has primarily relied on culture-based-methods and often suggested commensal bacteria (e.g. *Coagulase Negative Staphylococci,* CoNS) to play a role in HS. Although culture-based techniques have provided important insights into bacterial, fungal, and viral populations on the skin, their sensitivity is low. It is thought that the method cultures less than 1 % of the bacterial species present. Moreover, culture-based methods exclude microbes that rely on microbe-microbe interactions to thrive.

WO12112548 discloses *inter alia* that *Staphylococcus epidermidis* contributes to the skin resident microflora-based defense of the skin epithelium and that an imbalance of said microflora could contribute to the pathogenesis of skin inflammatory diseases, such as atopic dermatitis, rosacea and acne vulgaris etc. WO12112548 also discloses a method for treating an infection with Firmocidin, including infections caused by the presence of bacteria, such as dermatological disorder, including wounds, diabetic ulcers, acne, rosacea, atopic dermatitis, pyodermas, and burn wounds, by systemic or topical administration of an effective amount of a Firmocidin. WO12112548 concerns treatment of topical diseases with *Staphylococcus epidermidis-*derived antimicrobial agents (Firmocidin), in which said diseases are characterized by the presence - not the absence or abnormal distribution - of certain bacteria.

WO08103751 discloses that *Staphylococcus epidermidis* inhibit the growth of *Staphylococcus aureus* and Group A Streptococcus, the two leading causes of human skin infections and wound infections. WO08103751 also discloses that peptides derived from *Staphylococcus epidermidis* (delta-haemolysin and phenol soluble modulin-delta) may be used in topical or systemic treatment of infectious skin disorders. WO08103751 also discloses treatment of skin or systemic disorders, infectious or non-infectious, with topical application of delta-haemolysin, phenol soluble modulin-delta or a functional variant thereof would result in faster recovery from many dermatological diseases, including wounds, diabetic ulcers, acne, rosacea, atopic dermatitis, pyodermas, burn wounds, catheter infections, Group A Streptococcus, *Staphylococcus aureus* and other dermatological disease. WO08103751 concerns treatment of topical diseases with e.g. *Staphylococcus epidermidis,* in which said diseases are characterized by the *presence* - not the absence or abnormal distribution - of certain bacteria.

US2015051147 discloses *inter alia* topical treatment of Hidradenitis Suppurativa (HS) with an amnion-derived cellular cytokine solution (ACCS). The examples show *in vivo* experimental evidence on mice of the anti-inflammatory and wound healing effects of ACCS.

WO11153477 discloses treatment of Hidradenitis Suppurativa (HS) using TNFα inhibitors, such as the monoclonal IgG antibody, Adalimumab e.g. by use of subcutaneous administration.

The emergence of next-generation sequencing (NGS) has allowed a more accurate and less biased characterization of microbiomes compared to traditional culture-based techniques used in the prior art. Thus, it appears attractive to investigate the microbiome of HS using NGS thereby yielding a wider spectrum of bacterial species.

Hence, an improved method of identifying the microbiome of intertriginous areas in HS patients would be advantageous. In addition, such an improved method may additionally provide a useful diagnostic tool and provide important information concerning an effective method of treating HS.

### Summary of the invention

The inventors therefore designed a case-control study investigating the skin microbiome of healthy human subjects (i.e. human subjects not suffering from HS) and patients diagnosed with HS, using NGS targeting both 16S and 18S ribosomal RNA.

Insight into the cutaneous microbiome in HS using NGS provided hitherto unknown data on the microbiological diversity of the skin.

Thus, an object of the present invention relates to specifically defined saprophytic live bacteria originating from healthy human donors, i.e. human donors not suffering from HS, for use in an improved topical treatment of HS.

In particular, it is an object of the present invention to provide saprophytic live bacteria originating from healthy human donors that solves the above mentioned problems of the prior art resulting from an inadequate knowledge of the structure of the bacterial population as well as the diversity at strain level HS patients.

Thus, one aspect of the invention relates to saprophytic live bacteria mixture originating from the axillia of a human donor not suffering from Hidradenitis Suppurativa and who is having no previous record of dermatological diseases or other systemic diseases, which potentially could affect the cutaneous microbiome of the donor, for use in topical treatment of Hidradenitis Suppurativa in intertriginous skin areas of a human subject, wherein said saprophytic live bacteria mixture comprises at least the following: *Corynebacterium spp, Acinetobacter spp, Moraxella spp, Staphylococcus spp, Anaerococcus spp* and *Propionibacterium spp,*
wherein the *Propionibacterium spp* of the mixture is dominated by *Propionibacterium acnes* and with the proviso that the mixture does not contain *Porphyromonas* and *Peptoniphilus spp*

Still another aspect of the present invention relates to a method of diagnosing Hidradenitis Suppurativa (HS) in a human subject, wherein the method comprises at least the following consecutive steps:
(i) providing a biopsy sample that has been obtained by taking one or more skin biopsy sample(s) from intertriginous areas of said subject where at least one visible hair follicle is present,
(ii) extracting the DNA from the obtained biopsy sample(s),
(iii) amplifying and purifying the extracted DNA,
(iv) sequencing the amplified DNA by use of next-generation sequencing (NGS) targeting both 16S and 18S ribosomal RNA,
(v) based on the obtained NGS results, determine of the microbiome of bacteria contained in the biopsy sample(s), thereby
(vi) classifying the bacteria as *Corynebacterium spp, Acinetobacter spp* and *Moraxella spp, Staphylococcus spp* and *Anaerococcus spp, Porphyromonas* and *Peptoniphilus spp* and *Propionibacterium spp,*
thereby;
(1) if an absence of *Porphyromonas* and *Peptoniphilus spp* is observed this is an indication of skin not affected with HS, and
(2) if an absence of *Propionibacterium spp* is observed this is an indication of HS-affected skin, and
(3) if a predominance of *Porphyromonas* and *Peptoniphilus spp* is/are observed this is an indication of HS-affected skin.

### Brief description of the figures

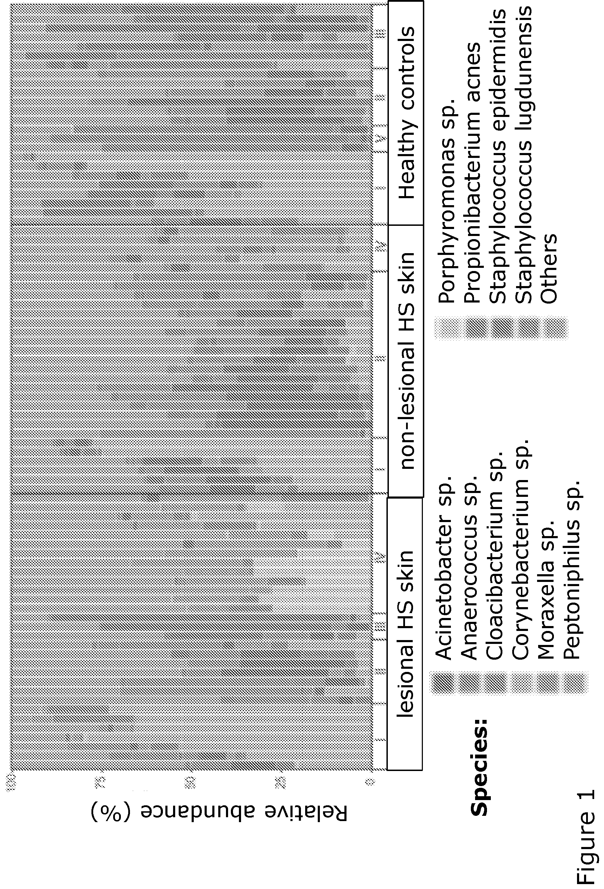

Figure 1 depicts the distribution of the top 10 most abundant species found in HS patients and healthy controls. Barplot showing the distribution of the 10 most abundant species found in lesional HS skin, non-lesional HS skin and healthy controls. 5 microbiome types were identified; *Corynebacterium spp* (of Type I), *Acinetobacter spp* and *Moraxella spp* (of Type II), *Staphylococcus spp (Staphylococcus epidermidis) and Anaerococcus spp* (of Type III), *Porphyromonas* and *Peptoniphilus spp* (of Type IV) and *Propionibacterium spp (P. acnes)* (of Type V).

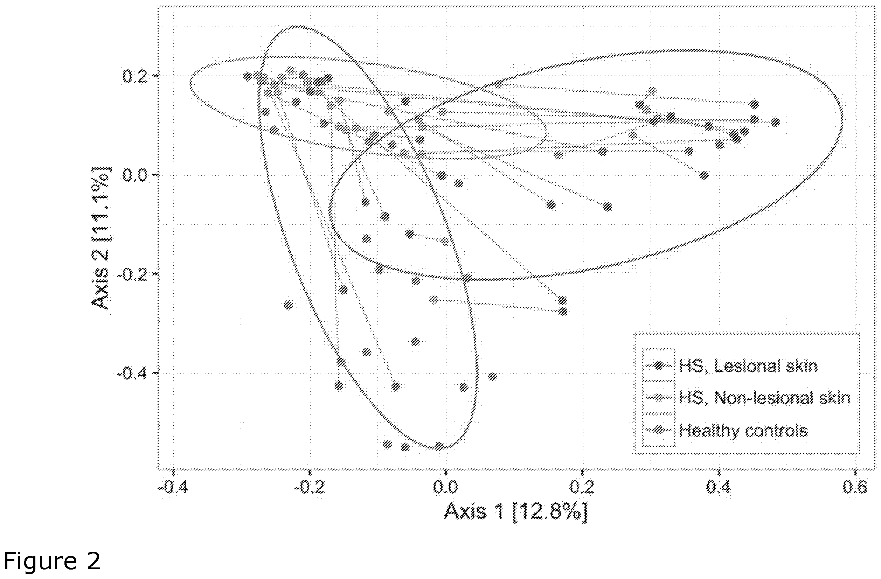

Figure 2 shows a PCoA plot illustrating differences between the three groups of subjects. More specifically, the PCoA plot illustrates the differences between the three groups (lesional, non-lesional and healthy controls). Each lesional sample is connected with its corresponding non-lesional sample. Ellipses delineate the 75% prediction areas of samples from each groups.

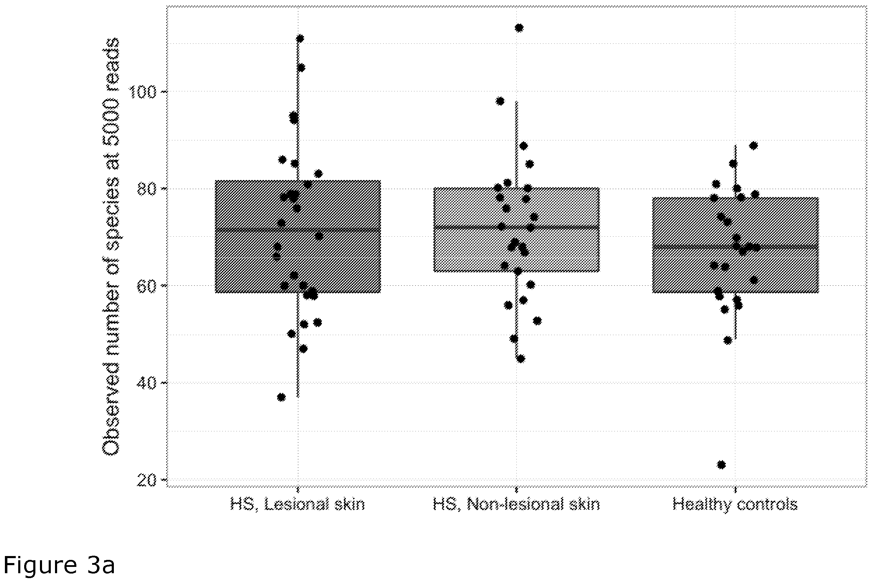

Figure 3a shows a Richness plot showing no difference between the number of species per sample in the groups (lesional, non-lesional and healthy controls, p = 0.66), after rarefaction to 5000 reads.

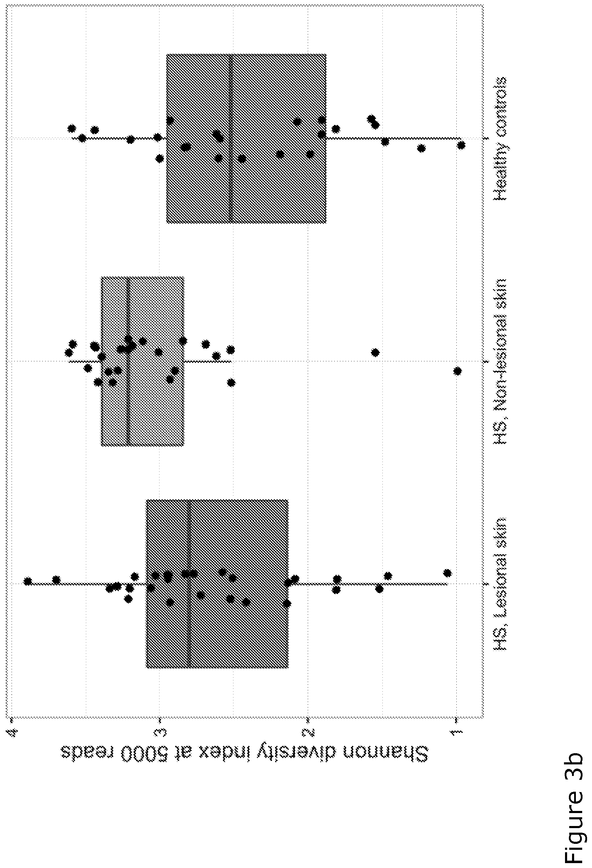

Figure 3b shows a Shannon index showing the diversity of the species in each group. The Shannon index reveals an increased diversity in the non-lesional skin group, compared to both lesional skin and healthy controls (p = 0.005).

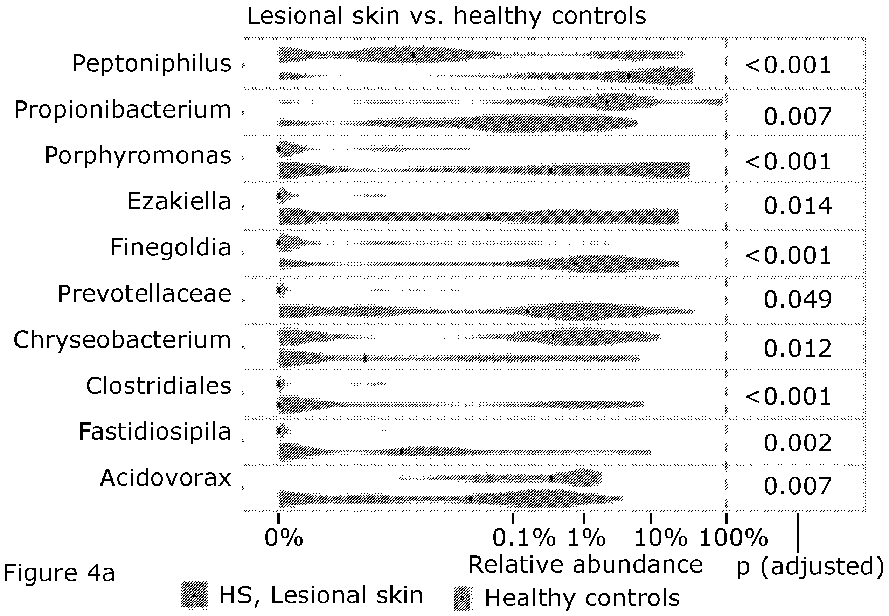

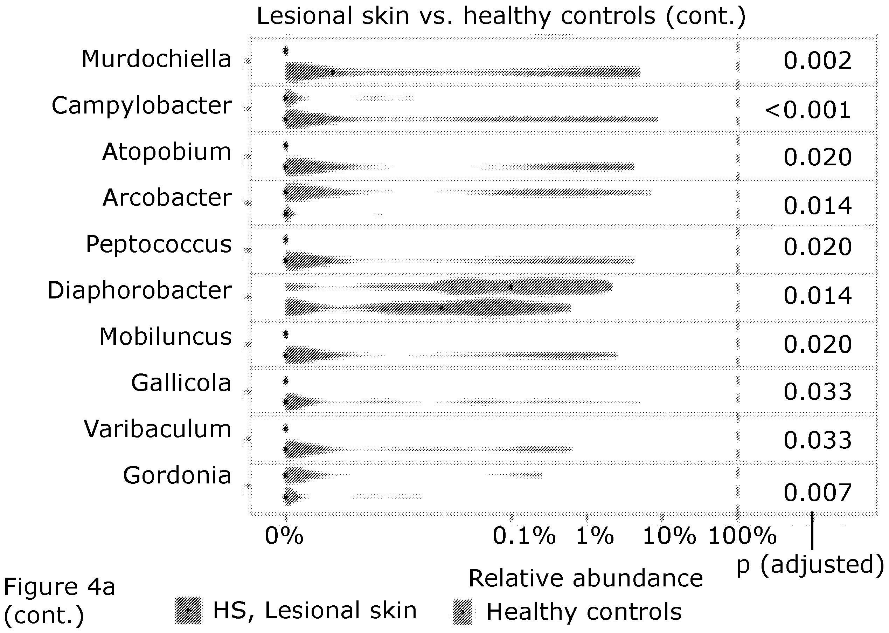

Figure 4a shows Differential Abundance (DA) at genus level between HS lesional skin and healthy controls. Note the significant higher relative abundance of Propionibacterium (p=0.007) found in healthy controls as well as the significant higher relative abundance of *Porphyromonas* (p<0.001) and *Peptoniphilus* (p<0.001). P-values listed were provided by the metagenomeSeq featureModel or Wilcoxon test. Black dots indicate medians of each distribution

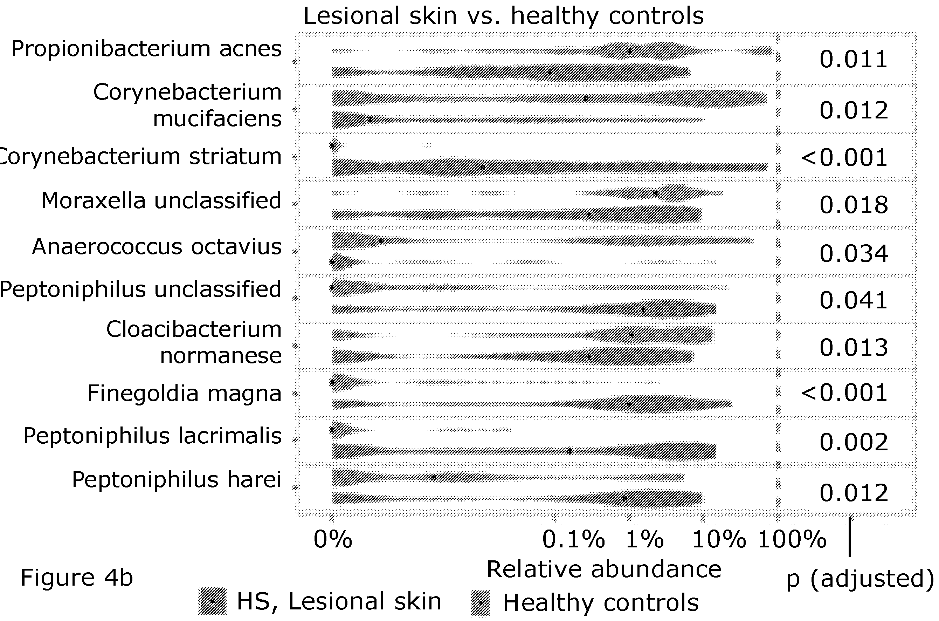

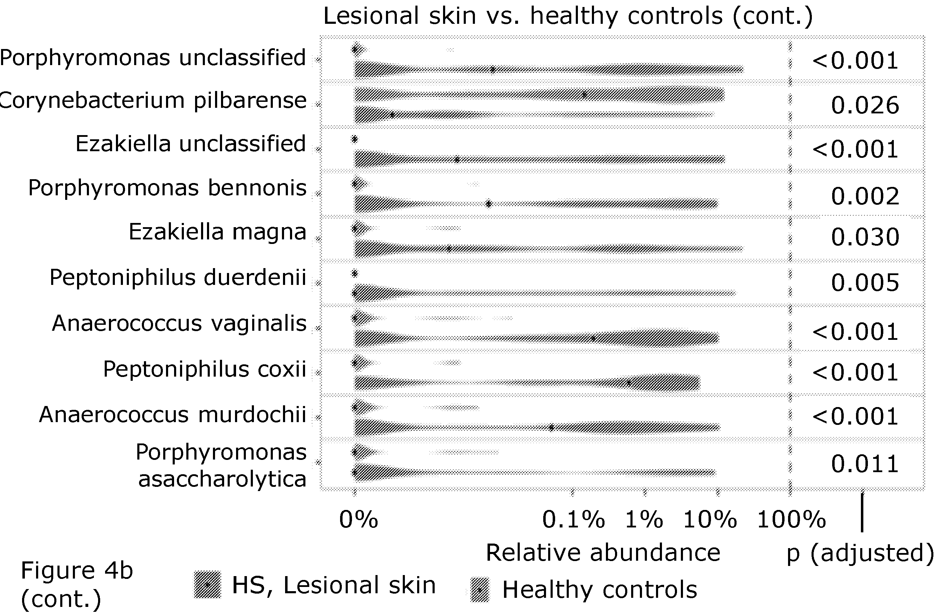

Figure 4b shows Differential Abundance (DA) at species level between HS lesional skin and healthy controls. *P. acnes* was found to have a higher relative abundance in healthy controls compared to that of lesional samples. P-values listed were provided by the metagenomeSeq featureModel or Wilcoxon test. Black dots indicate medians of each distribution.

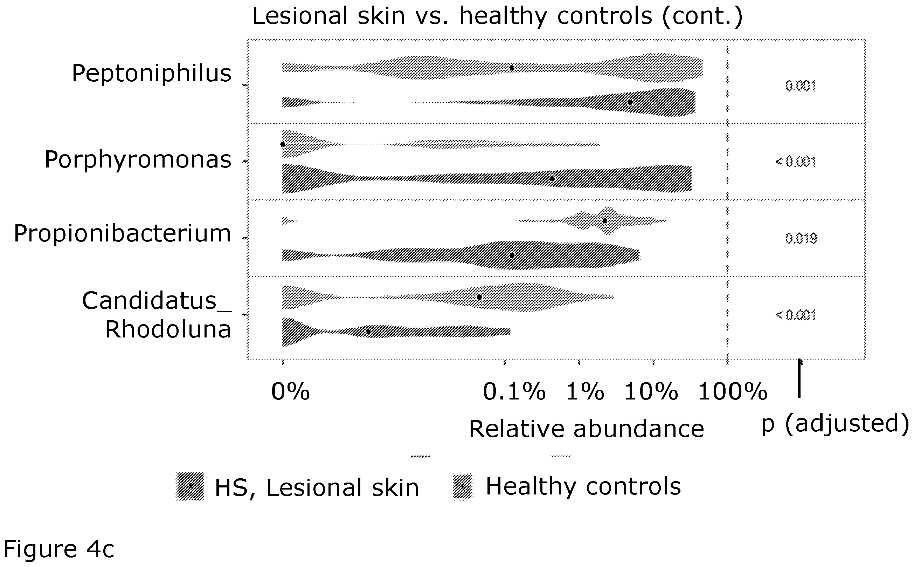

Figure 4c shows Differential Abundance (DA) at genus level between HS lesional skin and non-lesional HS skin. Note that *Porphyromonas* has a significant higher relative abundance in lesional skin than in non-lesional skin. P-values listed were provided by the metagenomeSeq featureModel or Wilcoxon test. Black dots indicate medians of each distribution.

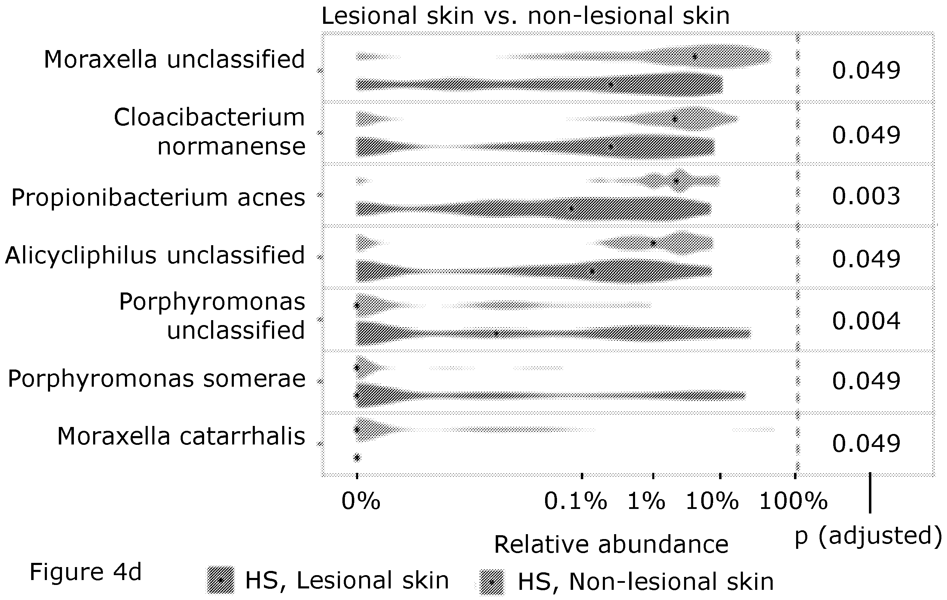

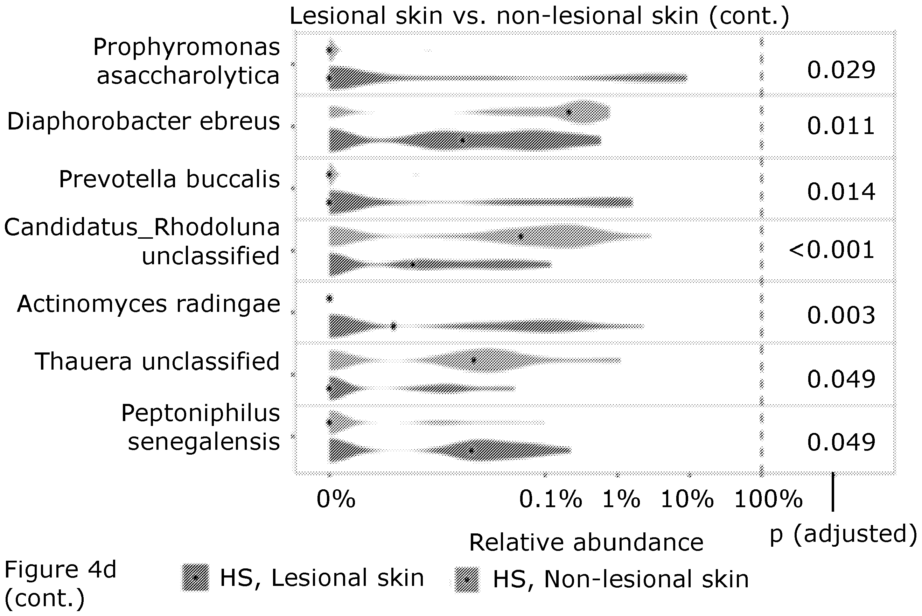

Figure 4d shows Differential Abundance (DA) at species level between HS lesional skin and non-lesional HS skin. As seen in figure 4b, a significantly reduced relative abundance of P. *acnes* is found in lesional HS skin and conversely a higher relative abundance of *Porphyromonas* is found in lesional skin. P-values listed were provided by Wilcoxon test. Black dots indicate medians of each distribution.
Figure 4e shows Differential Abundance (DA) at species level between HS non- lesional skin and healthy controls. Only four species were found statistical significant different between the non-lesional samples and the healthy controls. P-values listed were provided by Wilcoxon test. Black dots indicate medians of each distribution.

The present invention will now be described in more detail in the following.

### Detailed description of the invention

### Definitions

Prior to discussing the present invention in further details, the following terms and conventions will first be defined.

Bioinformatics: is an interdisciplinary field that develops methods and software tools for understanding biological data. As an interdisciplinary field of science, bioinformatics combines computer science, statistics, mathematics, and engineering to analyze and interpret biological data.

Colony-forming unit (CFU): is a unit used to estimate the number of viable bacteria or fungal cells in a sample.

Bray-Curtis distances (or dissimilarity): is a statistic used to quantify the compositional dissimilarity between two different sites, based on counts at each site. The Bray-Curtis dissimilarity is bound between 0 and 1, where 0 means the two sites have the same composition (that is they share all the species), and 1 means the two sites do not share any species. At sites with where BC is intermediate (e.g. BC = 0.5) this index differs from other commonly used indices.

Commensal microflora (normal microflora, indigenous microbiota): consists of those microorganisms, which are present on body surfaces covered by epithelial cells and are exposed to the external environment (gastrointestinal and respiratory tract, vagina, skin, etc.).
DA = Differential abundance
HS = Hidradenitis Suppurativa (the terms "HS" and "Hidradenitis Suppurativa" are used interchangeably throughout the application).
MALDI-TOF = Matrix-assisted laser desorption-time-of-flight.
Microbiome = While skin flora or skin microbiota refer to are the microorganisms which reside on the skin, the skin microbiome refers to the genomes of said microorganisms.
NGS = Next-generation Sequencing, which refers to non-Sanger-based high-throughput DNA sequencing technologies. Millions or billions of DNA strands can be sequenced in parallel, yielding substantially more throughput and minimizing the need for the fragment-cloning methods that are often used in Sanger sequencing of genomes.

Hurley staging system: is used to separate patients into three groups based largely on the presence and extent of cicatrization and sinus tracts. Stage I is a single lesion without sinus tract formation. Stage II manifests as more than one lesion or area, but with limited tunneling. Stage III is defined as multiple lesions, with more extensive sinus tracts and scarring.

PCoA plot: Principal Coordinates Analysis (PCoA) is a method to explore and to visualize dissimilarities of data. It starts with a dissimilarity matrix (= distance matrix, where distances can be calculated by using any number of tools, including Bray-Curtis) and assigns for each item a location in a low-dimensional space. PCoA tries to find the main axes through a matrix.

Saphrophyte: An organism, especially a fungus or bacterium that derives its nourishment from dead or decaying organic matter. Also called *saprobe.*

Sartorius score system: is a method for quantitating the severity of HS. This system also relies on physical findings, and allows calculation of a numerical score for each body area involved. Points are awarded for a) Involvement in specific body areas (3 points each body area), b) Nodules (2 points for each), c) Fistulas (4 points), d) Scars (1 point), e) Other findings (1 point), f) Longest distance between two lesions (2-4 points), g) If lesions are separated by normal skin (yes-0 points; No-6 points).

One aspect of the invention relates to saprophytic live bacteria mixture originating from the axillia of a human donor not suffering from Hidradenitis Suppurativa and who is having no previous record of dermatological diseases or other systemic diseases, which potentially could affect the cutaneous microbiome of the donor, for use in topical treatment of Hidradenitis Suppurativa in intertriginous skin areas of a human subject, wherein said saprophytic live bacteria mixture comprises at least the following: *Corynebacterium spp, Acinetobacter spp, Moraxella spp, Staphylococcus spp, Anaerococcus spp* and *Propionibacterium spp,*
wherein the *Propionibacterium spp* of the mixture is dominated by *Propionibacterium acnes* and with the proviso that the mixture does not contain *Porphyromonas* and *Peptoniphilus spp.*

Another aspect of the invention relates to saprophytic live bacteria mixture originating from the axillia of a human donor not suffering from Hidradenitis Suppurativa and who is having no previous record of dermatological diseases or other systemic diseases, which potentially could affect the cutaneous microbiome of the donor, for use in topical treatment of Hidradenitis Suppurativa in intertriginous skin areas of a human subject, wherein said saprophytic live bacteria mixture comprises at least the following: Corynebacterium spp, Acinetobacter spp, Moraxella spp, Staphylococcus spp, Anaerococcus spp and Propionibacterium spp,
wherein the Propionibacterium spp of the mixture is dominated by Propionibacterium acnes and with the proviso that the mixture does not contain Porphyromonas and Peptoniphilus sppwherein the *Staphylococcus spp* (of Type III) are selected from the group consisting of *Staphylococcus epidermidis, Staphylococcus deformans, Staphylococcus caprae* or *Staphylococcus capitis,* and
*Propionibacterium spp* (of Type V)

The present disclosure also relates to saprophytic live bacteria originating from a human donor not suffering from Hidradenitis Suppurativa for use in topical treatment of Hidradenitis Suppurativa in intertriginous skin areas of a human subject, wherein said saprophytic live bacteria comprises at least the following:
*Corynebacterium spp* (of Type I),
*Acinetobacter spp* and *Moraxella spp* (of Type II),
*Staphylococcus spp* and *Anaerococcus spp* (of Type III) and
*Propionibacterium spp* (of Type V)
wherein the bacteria of the *Propionibacterium spp* (of Type V) is *Propionibacterium acnes.*

The present disclosure also relates to saprophytic live bacteria originating from a human donor not suffering from Hidradenitis Suppurativa for use in topical treatment of Hidradenitis Suppurativa in intertriginous skin areas of a human subject, wherein said saprophytic live bacteria comprises at least the following:
*Corynebacterium spp* (of Type I),
*Acinetobacter spp* and *Moraxella spp* (of Type II),
*Staphylococcus spp* and *Anaerococcus spp* (of Type III) wherein the *Staphylococcus spp* (of Type III) are selected from the group consisting of *Staphylococcus epidermidis, Staphylococcus deformans, Staphylococcus caprae* or *Staphylococcus capitis,* and
*Propionibacterium spp* (of Type V) wherein the bacteria of the *Propionibacterium spp (of Type V) is Propionibacterium acnes.*

In another embodiment of the invention the intertriginous areas on which topical treatment of Hidradenitis Suppurativa shall be applied are the areas of the axilla or the groin.

The present disclosure relates to a saprophytic live bacteria originating from a human donor not suffering from Hidradenitis Suppurativa for use in topical treatment of
Hidradenitis Suppurativa in intertriginous skin areas of a human subject, wherein said saprophytic live bacteria comprises at least the following:
*Corynebacterium spp* (of Type I),
*Acinetobacter spp* and *Moraxella spp* (of Type II),
*Staphylococcus spp* and *Anaerococcus spp* (of Type III) and
*Propionibacterium spp* (of Type V) and
wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL.

The present disclosure also relates to saprophytic live bacteria originating from a human donor not suffering from Hidradenitis Suppurativa for use in topical treatment of Hidradenitis Suppurativa in intertriginous skin areas of a human subject, wherein said saprophytic live bacteria comprises at least the following:
*Corynebacterium spp* (of Type I),
*Acinetobacter spp* and *Moraxella spp* (of Type II),
*Staphylococcus spp* and *Anaerococcus spp* (of Type III) wherein the *Staphylococcus spp* (of Type III) are selected from the group consisting of *Staphylococcus epidermidis, Staphylococcus deformans, Staphylococcus caprae* or *Staphylococcus capitis,* and *Propionibacterium spp* (of Type V) and
wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL.

The present disclosure also relates to saprophytic live bacteria originating from a human donor not suffering from Hidradenitis Suppurativa for use in topical treatment of Hidradenitis Suppurativa in intertriginous skin areas of a human subject, wherein said saprophytic live bacteria comprises at least the following:
*Corynebacterium spp* (of Type I),
*Acinetobacter spp* and *Moraxella spp* (of Type II),
*Staphylococcus spp* and *Anaerococcus spp* (of Type III) and
*Propionibacterium spp* (of Type V) wherein the bacteria of the *Propionibacterium spp* (of Type V) is *Propionibacterium acnes* and
wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL..

The present disclosure also relates to saprophytic live bacteria originating from a human donor not suffering from Hidradenitis Suppurativa for use in topical treatment of
Hidradenitis Suppurativa in intertriginous skin areas of a human subject, wherein said saprophytic live bacteria comprises at least the following:
*Corynebacterium spp* (of Type I),
*Acinetobacter spp* and *Moraxella spp* (of Type II),
*Staphylococcus spp* and *Anaerococcus spp* (of Type III) wherein the *Staphylococcus spp* (of Type III) are selected from the group consisting of *Staphylococcus epidermidis, Staphylococcus deformans, Staphylococcus caprae* or *Staphylococcus capitis,*
   and
*Propionibacterium spp* (of Type V) wherein the bacteria of the *Propionibacterium spp (of Type V) is Propionibacterium acnes* and
wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL.

In another embodiment of the invention the bacteria are topically applied directly to the skin via sprays, roll-on deodorants, creams, fats, pastes, foams, gels, lotions or ointments.

Still another aspect of the invention relates to a saprophytic live bacteria mixture originating from the axillia of a human donor not suffering from Hidradenitis Suppurativa and who is having no previous record of dermatological diseases or other systemic diseases, which potentially could affect the cutaneous microbiome of the donor, for use in topical treatment of Hidradenitis Suppurativa in intertriginous skin areas of a human subject, wherein said saprophytic live bacteria mixture comprises at least the following:
*Corynebacterium spp, Acinetobacter spp, Moraxella spp, Staphylococcus spp,*
*Anaerococcus spp* and *Propionibacterium spp,*
wherein the *Propionibacterium spp* of the mixture is dominated by *Propionibacterium acnes* and with the proviso that the mixture does not contain *Porphyromonas* and *Peptoniphilus* sppwherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

The present disclosure also relates to saprophytic live bacteria originating from a human donor not suffering from Hidradenitis Suppurativa for use in topical treatment of Hidradenitis Suppurativa in intertriginous skin areas of a human subject, wherein said saprophytic live bacteria comprises at least the following:
*Corynebacterium spp* (of Type I),
*Acinetobacter spp* and *Moraxella spp* (of Type II),
*Staphylococcus spp* and *Anaerococcus spp* (of Type III) wherein the *Staphylococcus spp* (of Type III) are selected from the group consisting of *Staphylococcus epidermidis,*
*Staphylococcus deformans, Staphylococcus caprae* or *Staphylococcus capitis,* and *Propionibacterium spp* (of Type V) and
wherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

The present disclosure also relates to saprophytic live bacteria originating from a human donor not suffering from Hidradenitis Suppurativa for use in topical treatment of Hidradenitis Suppurativa in intertriginous skin areas of a human subject, wherein said saprophytic live bacteria comprises at least the following:
*Corynebacterium spp* (of Type I),
*Acinetobacter spp* and *Moraxella spp* (of Type II),
*Staphylococcus spp* and *Anaerococcus spp* (of Type III) and
*Propionibacterium spp* (of Type V)
wherein the bacteria of the *Propionibacterium spp* (of Type V) is *Propionibacterium acnes.*

The present disclosure also relates to saprophytic live bacteria originating from a human donor not suffering from Hidradenitis Suppurativa for use in topical treatment of Hidradenitis Suppurativa in intertriginous skin areas of a human subject, wherein said saprophytic live bacteria comprises at least the following:
*Corynebacterium spp* (of Type I),
*Acinetobacter spp* and *Moraxella spp* (of Type II),
*Staphylococcus spp* and *Anaerococcus spp* (of Type III) and
*Propionibacterium spp* (of Type V) and
wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL and wherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

The present disclosure also relates to saprophytic live bacteria originating from a human donor not suffering from Hidradenitis Suppurativa for use in topical treatment of Hidradenitis Suppurativa in intertriginous skin areas of a human subject, wherein said saprophytic live bacteria comprises at least the following:
*Corynebacterium spp* (of Type I),
*Acinetobacter spp* and *Moraxella spp* (of Type II),
*Staphylococcus spp* and *Anaerococcus spp* (of Type III) wherein the *Staphylococcus spp* (of Type III) are selected from the group consisting of *Staphylococcus epidermidis, Staphylococcus deformans, Staphylococcus caprae* or *Staphylococcus capitis,* and
*Propionibacterium spp* (of Type V) and
wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL and wherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

The present disclosure also relates to saprophytic live bacteria originating from a human donor not suffering from Hidradenitis Suppurativa for use in topical treatment of Hidradenitis Suppurativa in intertriginous skin areas of a human subject, wherein said saprophytic live bacteria comprises at least the following:
*Corynebacterium spp* (of Type I),
*Acinetobacter spp* and *Moraxella spp* (of Type II),
*Staphylococcus spp* and *Anaerococcus spp* (of Type III) and
*Propionibacterium spp* (of Type V) wherein the bacteria of the *Propionibacterium spp* (of Type V) is *Propionibacterium acnes* and
wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL and wherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

The present disclosure also relates to saprophytic live bacteria originating from a human donor not suffering from Hidradenitis Suppurativa for use in topical treatment of Hidradenitis Suppurativa in intertriginous skin areas of a human subject, wherein said saprophytic live bacteria comprises at least the following:
*Corynebacterium spp* (of Type I),
*Acinetobacter spp* and *Moraxella spp* (of Type II),
*Staphylococcus spp* and *Anaerococcus spp* (of Type III) wherein the *Staphylococcus spp* (of Type III) are selected from the group consisting of *Staphylococcus epidermidis, Staphylococcus deformans, Staphylococcus caprae* or *Staphylococcus capitis,*
   and
*Propionibacterium spp* (of Type V) wherein the bacteria of the *Propionibacterium spp (of Type V) is Propionibacterium acnes* and
wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL and
wherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

In another embodiment of the invention the period between consecutive topical treatments of Hidradenitis Suppurativa with the saprophytic bacteria of the invention in intertriginous skin areas of a human subject is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

Still another aspect of the invention relates to saprophytic live bacteria originating from a human donor not suffering from Hidradenitis Suppurativa for use in topical treatment of Hidradenitis Suppurativa in intertriginous skin areas of a female human subject.

Still another aspect of the invention relates to saprophytic live bacteria originating from a human donor not suffering from Hidradenitis Suppurativa for use in topical treatment of Hidradenitis Suppurativa in intertriginous skin areas of a male human subject.

Still another aspect of the invention relates to saprophytic live bacteria originating from a human donor not suffering from Hidradenitis Suppurativa for use in topical treatment of Hidradenitis Suppurativa in intertriginous skin areas of a human subject wherein said bacteria are topically applied to the human subject as a mixture of said bacteria.

Still another aspect of the invention relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)

- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).
wherein the *Staphylococcus spp* (of Type III) are selected from the group consisting of *Staphylococcus epidermidis, Staphylococcus deformans, Staphylococcus caprae* or *Staphylococcus capitis.*

The present invention also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp (of Type II)*: *5-95* %, *such as 10-90* %, *such as 20-80* %, *such as 30-70* %, *such as 40-60* %, *such as 50* % *CFU of Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).
wherein the bacteria of the *Propionibacterium spp* (of Type V) is *Propionibacterium acnes.*

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).
wherein the *Staphylococcus spp* (of Type III) are selected from the group consisting of *Staphylococcus epidermidis, Staphylococcus deformans, Staphylococcus caprae* or *Staphylococcus capitis* and wherein the bacteria of the *Propionibacterium spp (of Type V) is Propionibacterium acnes.*

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).
and wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL.

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).
wherein the *Staphylococcus spp* (of Type III) are selected from the group consisting of *Staphylococcus epidermidis, Staphylococcus deformans, Staphylococcus caprae* or *Staphylococcus capitis* and wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL.

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).
wherein the bacteria of the *Propionibacterium spp* (of Type V) is *Propionibacterium acnes* and wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL.

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).
wherein the *Staphylococcus spp* (of Type III) are selected from the group consisting of *Staphylococcus epidermidis, Staphylococcus deformans, Staphylococcus caprae* or *Staphylococcus capitis* wherein the bacteria of the *Propionibacterium spp (of Type V) is Propionibacterium acnes* and wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL.

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V)
wherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).
wherein the *Staphylococcus spp* (of Type III) are selected from the group consisting of *Staphylococcus epidermidis, Staphylococcus deformans, Staphylococcus caprae* or *Staphylococcus capitis,* and wherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V)
wherein the bacteria of the *Propionibacterium spp* (of Type V) is *Propionibacterium acnes.*

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).
wherein the *Staphylococcus spp* (of Type III) are selected from the group consisting of *Staphylococcus epidermidis, Staphylococcus deformans, Staphylococcus caprae* or *Staphylococcus capitis and* wherein the bacteria of the *Propionibacterium spp (of Type V) is Propionibacterium acnes* and wherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).
and wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL and wherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),

- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).
wherein the *Staphylococcus spp* (of Type III) are selected from the group consisting of *Staphylococcus epidermidis, Staphylococcus deformans, Staphylococcus caprae* or *Staphylococcus capitis,* and wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL and wherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),

- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V)
wherein the bacteria of the *Propionibacterium spp* (of Type V) is *Propionibacterium acnes* and wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL and wherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

The present disclosure also relates to a mixture of saprophytic live bacteria originating from a human donor not suffering from Hidradenitis Suppurativa for use in topical treatment of Hidradenitis Suppurativa in intertriginous skin areas of a human subject, wherein said mixture comprises at least the following percentage ratios of bacterial CFU:
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).
wherein the *Staphylococcus spp* (of Type III) are selected from the group consisting of *Staphylococcus epidermidis, Staphylococcus deformans, Staphylococcus caprae* or *Staphylococcus capitis* and wherein the bacteria of the *Propionibacterium spp (of Type V) is Propionibacterium acnes* and wherein the concentrations of the topically applied bacteria are between 1-10⁹ bacteria/mL, such as between 10²-10⁹ bacteria/mL, such as between 10³-10⁸ bacteria/mL, such as between 10⁴-10⁷ bacteria/mL, such as between 10⁵-10⁶ bacteria/mL and wherein the period of treatment is between 2-180 days, such as 5-170 days, such as 10-160 days, such as 20-150 days, such as 30-140 days, such as 40-130 days, such as 50-120 days, such as 60-110 days, such as 70-100 days, such as 80-90 days.

The present disclosure also relates to saprophytic live bacteria or mixture of saprophytic live bacteria originating from a human donor not suffering from Hidradenitis Suppurativa for use in topical treatment of Hidradenitis Suppurativa in the intertriginous skin areas of a human subject, wherein said saprophytic live bacteria or mixture comprises the following percentage ratios of bacterial CFU:
- 5-95 % CFU of *Corynebacterium spp* (of Type I): 5-95 % CFU *Acinetobacter spp* and *Moraxella spp* (of Type II),
- 5-95 % CFU of *Corynebacterium spp* (of Type I): 5-95 % CFU of *Staphylococcus spp* and *Anaerococcus spp* (of Type III),
- 5-95 % CFU of *Corynebacterium spp* (of Type I): 5-95 % CFU of *Propionibacterium spp* (of Type V)
- 5-95 % CFU of *Acinetobacter spp* and *Moraxella spp* (of Type II): 5-95 % CFU of *Staphylococcus spp* and *Anaerococcus spp* (of Type III)
- 5-95 % CFU of *Acinetobacter spp* and *Moraxella spp* (of Type II): 5-95 % CFU of *Propionibacterium spp* (of Type V)
- 5-95 % CFU of *Staphylococcus spp* and *Anaerococcus spp* (of Type III): 5-95 % CFU of *Propionibacterium spp* (of Type V).
where the concentrations of topically applied bacteria are between 1-10⁹ bacteria/mL culture medium and the period of treatment is between 2-180 days.

The present disclosure also relates to a method of predicting the chances of curative success following topical treatment of Hidradenitis Suppurativa in a human subject with any of the following saprophytic live bacteria: *Corynebacterium spp* (of Type I), *Acinetobacter spp* and *Moraxella spp* (of Type II), *Staphylococcus spp* and *Anaerococcus spp* (of Type III), *Porphyromonas* and *Peptoniphilus spp* (of Type IV) and *Propionibacterium spp* (of Type V),
wherein the method comprises at least the following consecutive steps:
(i) obtaining one or more skin biopsy sample(s) from intertriginous areas of the human subject where at least one visible hair follicle is present
(ii) extracting the DNA from the obtained biopsy sample(s)
(iii) amplifying and purifying the extracted DNA,
(iv) sequencing the amplified DNA by use of next-generation sequencing (NGS) targeting both 16S and 18S ribosomal RNA,
(v) based on the obtained NGS results, determine of the microbiome of bacteria contained in the biopsy sample(s), thereby
(vi) classifying the bacteria as *Corynebacterium spp* (of Type I), *Acinetobacter spp* and *Moraxella spp* (of Type II), *Staphylococcus spp* and *Anaerococcus spp* (of Type III), *Porphyromonas* and *Peptoniphilus spp* (of Type IV) and *Propionibacterium spp* (of Type V),
thereby;
(1) based on the composition of Type I-V bacteria of the biopsy sample(s) of the human subject, predicting the chances of curative success.

Still another aspect of the invention relates to a method of diagnosing Hidradenitis Suppurativa (HS) in a human subject, wherein the method comprises at least the following consecutive steps:
(i) providing a biopsy sample that has been obtained by taking one or more skin biopsy sample(s) from intertriginous areas of said subject where at least one visible hair follicle is present,
(ii) extracting the DNA from the obtained biopsy sample(s),
(iii) amplifying and purifying the extracted DNA,
(iv) sequencing the amplified DNA by use of next-generation sequencing (NGS) targeting both 16S and 18S ribosomal RNA,
(v) based on the obtained NGS results, determine of the microbiome of bacteria contained in the biopsy sample(s), thereby
(vi) classifying the bacteria as *Corynebacterium spp* (of Type I), *Acinetobacter spp* and *Moraxella spp* (of Type II), *Staphylococcus spp* and *Anaerococcus spp* (of Type III), *Porphyromonas* and *Peptoniphilus spp* (of Type IV) and *Propionibacterium spp* (of Type V),
thereby;
(1) if an absence of *Porphyromonas* and *Peptoniphilus spp* (of Type IV) is observed this is an indication of skin not affected with HS, and
(2) if an absence of *Propionibacterium spp* (of Type V) is observed this is an indication of HS-affected skin, and
(3) if a predominance of *Porphyromonas* and *Peptoniphilus spp* (of Type IV) is/are observed this is an indication of HS-affected skin.

It should be noted that embodiments and features described in the context of one of the aspects of the present invention also apply to the other aspects of the invention.

The present disclosure also relates to a method of treating Hidradenitis Suppurativa (HS) in a human subject by applying the following percentage ratios of saprophytic live bacteria or a mixture of saprophytic live bacteria originating from a human donor not suffering from Hidradenitis Suppurativa to intertriginous skin areas of a human subject::
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU *Acinetobacter spp* and *Moraxella spp* (of Type II),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Staphylococcus spp* and *Anaerococcus spp* (of Type III),
- 5-95 %, such as 10-90 %, such as 20-80 %, such as 30-70 %, such as 40-60 %, such as 50 % CFU of *Corynebacterium spp* (of Type I): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Staphylococcus spp* and *Anaerococcus spp* (of Type III)
- 5-95 %, such as 10-90 %, such as 20-80 %, such as 30-70 %, such as 40-60 %, such as 50 % CFU of *Acinetobacter spp* and *Moraxella spp* (of Type II): 5-95 %, such as 10-90 %, such as 20-80 %, such as 30-70 %, such as 40-60 %, such as 50 % CFU of *Propionibacterium spp* (of Type V)
- 5-95 %, such as 10-90 %, such as 20-80 %, such as 30-70 %, such as 40-60 %, such as 50 % CFU of *Staphylococcus spp* and *Anaerococcus spp* (of Type III): 5-95 %, such as 10-90 %, such as 20-80 %, such as 30-70 %, such as 40-60 %, such as 50 % CFU of *Propionibacterium spp* (of Type V).

The invention will now be described in further details in the following non-limiting examples.

### Examples

### Example 1

### Study Design

An exploratory case-control study comparing individuals (patients) with the diagnosis of HS (n=30) with healthy controls (n=24).

### Study Participants

All cases (patients) had a verified diagnosis of HS. In order to verify the diagnosis of HS histological, biopsies from lesional skin were obtained among all included patients. Furthermore, all patients were above 18 years of age, non-pregnant, and experienced a flare of at least one inflamed nodule located either in the axilla or in the groin.

### Table 1

**Table 1. Metadata displaying the background factors and characteristics. HS = Hidradenitis Suppurativa. SD = Standard deviation. BMI = Body mass index.**

| Characteristic | HS patients | Healthy controls |
|---|---|---|
| Total subjects analysed | 30 | 24 |
| Age in years (SD) | 46.9 (14.0) | 32 (12.0) |
| Female: Male [%] | 63:37 | 50:50 |
| BMI (SD) | 30 (7.7) | 26 (2.59) |
| Smoking status [%] | 68 | - |
| Sartorius score (SD) | 24 (20.0) | - |
| Ethnicity; Caucasian [%] | 100 | 96 |
| HS Severity (Hurley stage 1) [%] | 0 | - |
| HS Severity (Hurley stage 2) [%] | 87 | - |
| HS Severity (Hurley stage 3) [%] | 23 | - |
| Duration of lesions, days (median [IQR]) | 21 [14-60] | - |
| Location of Lesion | Groin (n=15), Axilla (n=15) | - |
| Diameter of lesions (SD) | 2.76 (1.38) | |

### HS Severity

An experienced dermatologist assessed patients during physical examination using Hurley staging and Sartorius score. Both systems rely on physical findings such as involvement of specific anatomical sites, number of nodules, sinus tracts and scars (Freysz et al., 2015;Zouboulis et al., 2015a). The Hurley system is a classification of three levels (mild (I), moderate (II) and severe (III)) (Zouboulis et al., 2015b). The Sartorius system allows calculation of a numerical score for each body area involved and points are awarded for each skin manifestation with no upper limit (Sartorius et al., 2009). The diameter for all nodules were measured and all patients reported the duration of the lesion (how long time the patients has noticed the presence of the lesion)

### Skin Biopsies

Prior to injection of anesthetics, the skin was cleansed with ethanol swabs (FastCare®, 70% Isopropyl Alcohol, Zhejian, China) to minimize subsequent skin infection. Lesional skin and non-lesional HS skin was anesthetized using a solution of lidocaine and adrenalin (lidocainhydrochlorid 20 mg/ml, Adrenalin 5 µg/ml, no preservatives). In HS patients, one 4 mm punch biopsy was obtained from lesional skin (axilla or groin) and non-lesional skin, respectively. Biopsies from the non-lesional HS skin was obtained approximately 5 cm for the inflamed nodule and was clinically unaffected (without scarring, inflammation or swelling).

The non-lesional biopsies were not checked for histological hallmarks compatible with early pathological changes of HS lesions. Moreover, the inventors did not obtain information on whether HS patients previously had experienced disease activity in the non-lesional area. However, as the non-lesional biopsies where all obtained in intertriginous areas (axilla or groin) and only 5 cm from the lesion, the samples most likely represent sites where HS lesions eventually will occur. Only nodules containing at least one visible hair follicle were biopsied. Biopsies from healthy controls also contained clinically visible hair follicles. Biopsies from healthy controls were obtained from the axilla only. Biopsy was performed using sterile gloves and the tissue was immediately placed into a sterile Eppendorf Safe-Lock tube and stored at -80 degrees. The cells were lysed within 48 hours.

### Table 2

**Table 2: Location of lesion and the corresponding microbiome types. N = Number of participants, HS = Hidradenitis Suppurativa.**

| Microbiome type | N | Location of lesion | |
|---|---|---|---|
| HS patients (n=30, lesional) | | Groin | Axilla |
| I | 7 | 3 | 4 |
| II | 7 | 1 | 6 |
| III | 3 | 1 | 2 |
| IV | 13 | 10 | 3 |
| V | 0 | 0 | 0 |

| HS patients (n=29, non-lesional) | | | |
|---|---|---|---|
| I | 6 | 3 | 3 |
| II | 18 | 6 | 12 |
| III | 0 | 0 | 0 |
| IV | 5 | 5 | 0 |
| V | 0 | 0 | 0 |

| Healthy controls (n=24) | | | |
|---|---|---|---|
| I | 8 | - | 8 |
| II | 6 | - | 6 |
| III | 7 | - | 7 |
| IV | 0 | - | 0 |
| V | 3 | - | 3 |

### Microbiome sequencing and analysis

DNA from the obtained biopsies was extracted using QIAamp® DNA mini Kit (Qiagen, Hilden, Germany) according to manufacture instruction for tissues. For each batch of DNA extraction, a "negative" control was included containing buffers but no sample material for downstream analysis.

DNA was amplified using a two-step PCR using custom 341F/806R primers targeting the V3-V4 16S regions, and 3 primer sets targeting the hyper-variable regions V3-V4 of the 18S rDNA gene, and amplicons were sequenced on the Illumina MiSeq desktop sequencer (Illumina Inc., San Diego, CA 29122, USA), using the V2 Reagent Kit. Sequence data is available at European Nucleotide Archive (accession number, PRJEB15266).

### Microbiome sequencing and analysis - Primer design (for use in NGS)

The 16S rDNA gene was targeted for amplification, using a modified version of the published universal prokaryotic primers 341F/806R, targeting the V3-V4 hyper-variable regions (pmid 15696537). The forward primer had three additional nucleotides attached in the 5' end (ACTCCTAYGGGRBGCASCAG, 341F3) and the reverse primer had five additional nucleotides attached in the 5' end (AGCGTGGACTACNNGGGTATCTAAT, 806R5).

The 18S rDNA gene was selected as target gene to ensure an as broad spectrum of eukaryotic species (parasites and fungi) to be amplified from as few primer sets as possible, when assuming that the 18S rDNA sequence would be the most inter-species conserved gene. 18S rDNA sequences were aligned for all species in each of 56 parasite genus' and a consensus sequence was generated. The consensus sequences for each genus were used for phylogenetic analysis in order to group the parasites according to their 18S rDNA sequence similarity rather than taxonomy. Six distinct groups appeared, and the consensus sequence from all species within each group was aligned and primers were designed, to amplify the species within each group. Additionally, a consensus sequence from human 18S rDNA was compared to the alignment in order to design primers that would avoid human 18S rDNA.

Three different primer sets were chosen, G3F1/G3R1 (GCCAGCAGCCGCGGTAATTC / ACATTCTTGGCAAATGCTTTCGCAG), G4F3/G4R3 (CAGCCGCGGTAATTCCAGCTC / GGTGGTGCCCTTCCGTCAAT) and G6F1/G6R1 (TGGAGGGCAAGTCTGGTGCC / ACGGTATCTGATCGTCTTCGATCCC). G3 and G6 primers are targeting the hyper-variable regions V3-V4 of the 18S rDNA gene, and G4 is targeting V3-V5. Each primer set was aligned using BLAST to the NCBI database, using NCBI's Primer-Blast, with standard settings (excluding predicted Refseq transcripts and uncultured / environmental samples) to test for unintended amplification.

### Microbiome sequencing and analysis - Library preparation

Purified genomic DNA from each sample was initially amplified using the 16S and 18S primers. The 16S and 18S rDNA was amplified in a 25µl volume, using the REDExtract-N-Amp PCR ReadyMix (Sigma-Aldrich, St Louis, MO, USA) with 0.4µM of each primer and 2µl template. The 16S PCR was run with an initial denaturation at 95°C for 2 min, 20 cycles of 95°C for 30 sec, 60°C for 1 min, and 72°C for 30 sec, and a final elongation at 72°C for 7 min. The 18S PCR setup was run with an initial denaturation at 95°C for 3 min, 20 cycles of 95°C for 1 min, 60°C for 1 min, and 72°C for 30 sec, and a final elongation at 72°C for 4 min.

These PCR runs are referred to at PCR1 or amplification PCR. The products from PCR1 were prepared for sequencing by a second PCR (PCR2 or adaptor PCR), using the same PCR protocol as described above. PCR2 attached an adaptor A, an index i5, and a forward sequencing primer site (FSP) in the 5' end of the amplicons and an adaptor B, an index i7, and a reverse sequencing primer site (RSP) to the 3' end of the amplicons. DNA was quantified using the Quant-ITTM dsDNA High Sensitive Assay Kit (Thermo Fisher Scientific), and PCR2 products were pooled in equimolar amounts between samples.

Undesirable DNA amplicons were removed from the pooled amplicon library (PAL) by Agencourt AMPure XP bead (Beckman Coulter) purification in a two-step process. Firstly, DNA fragments below 300nt were removed by a PAL AMPure beads 10:24 ratio, following the manufactures protocol and eluted in 40µL TE buffer (AM1). Secondly, large DNA fragments above 1kbp were removed by AM1 to AMPure beads 10:16 ratio, as previously described.

The resulting AMPure beads purified PAL (bPAL) was diluted to its final concentration of 11.5pM DNA in a 0.001 N NaOH, used for sequencing on the Illumina MiSeq desktop sequencer (Illumina Inc., San Diego, CA 29122, USA). The library was sequenced with the 500-cycle MiSeq Reagent Kit V2 in a 2x250nt setup (Illumina Inc., San Diego, CA 29122, USA).

### Bioinformatics

BION (http://box.com/bion), a newly developed analytical semi-commercial open-source package for 16S rRNA and other reference gene analysis, classifying mostly to species was used. The pipeline accepts raw sequence and includes steps for de-multiplexing, primer-extraction, sampling, sequence- and quality based trimming and filtering, de-replication, clustering, chimera-checking, reference data similarities and taxonomic mapping and formatting. Non-overlapping paired reads are allowed for analysis, and BION is often accurate to the species level.

### Statistics and data analysis

All data analysis was conducted using the statistical software package R v. 3.2.3 (R Core Team and R Foundation for Statistical Computing, 2015). Microbiome data was handled using the add-on package 'phyloseq' v. 1.16.2 (McMurdie and Holmes, 2013) and visualized with 'ggplot2' v. 2.1.0 (Wickham H, 2009). In the barplot, species belonging to the genera Staphylococcus and Propionibacterium were analyzed on species level, all other species were merged to genus level, by agglomerating counts within each genus. Counts were normalized to percentages per sample and the ten species with the highest sum of percentage were kept.
Microbiome types were defined using vegdist (from the add-on package 'vegan', v. 2.3-2) (Oksanen J et al., 2015) and hierarchal clustering with default settings, the number of optimal groups was decided by manual inspection.

Differences between groups were assessed with barplots and Principal Coordinates Analysis (PCoA) plots using Bray-Curtis distances and tested with a permutational multivariate analysis of variance (PERMANOVA), 'adonis' from the package 'vegan'.

Differential abundances were visualized with violin plots of proportional abundances on a log scale using a pseudocount of 10-6 and tested using 'fitFeatureModel' from the package 'metagenomeSeq' v. 1.14.2 (Paulson et al., 2013) with default settings. For taxa with less than 2 positive samples in one group, where the model failed, a Wilcoxon rank-sum test was used instead. This was conducted at species level, using the species defined by the BION pipeline, as well as at genus level, by agglomerating counts within each genus. P-values were adjusted for false discovery rates using the Benjamini-Hochberg approach (Benjamini Y and Hochberg Y, 1995).

Distributions of observed number of species and Shannon index between sample groups were visualized with boxplots and tested using Kruskal-Wallis tests (between the 3 groups) or Wilcoxon tests (pairwise comparisons) after bootstrap rarefaction to equal library size (5000 reads), repeated 100 times with the median value used. Statistical analyses were performed for associations between the five microbiome types in the three groups and the variables BMI, Sartorius, Hurley Stage, smoking, age and gender using Fisher's exact test for categorical variables and Kruskal-Wallis test for continuous variables. A p-value below 0.05 was considered statistically significant.

### Results

The cutaneous microbiome was characterized in 30 HS patients and 24 healthy controls. In total, 83 punch biopsies were obtained from HS patients and healthy controls (30 HS lesional (group 1), 29 non-lesional (group 2) and 24 healthy control samples (group 3), respectively).

Background factors and characteristics were as displayed in table 1 showing that a majority of the HS patients were predominantly younger, female and smokers. Overall, the data demonstrated that the microbiome in inflamed HS nodules, non-lesional skin as well as in samples from healthy controls differed significantly from each other (Fig. 1, Fig. 2 and table 2).

The barplot in Fig.1 depicts the immediate differences in the bacterial community with the 10 most abundant species or genera across the three groups. Several different unique types (I to V) of the skin microbiome were defined using hierarchical clustering of Bray-Curtis distances (Fig.1).

The microbiome types in lesional skin consisted predominantly of *Corynebacterium spp* (Type I) or *Porphyromonas* and *Peptoniphilus spp* (Type IV), respectively. In contrast, *Acinetobacter sp* and *Moraxella sp* (Type II) dominated non-lesional skin. In healthy controls, the various microbiome types (I, II, III and V) were more equally distributed.

Microbiome types III and V were dominated by *Staphylococcus epidermidis (S. epidermidis)* and *Propionibacterium acnes (P. acnes),* respectively. The microbiome type IV, was not found in healthy controls and microbiome type V was not found in either lesional - or non-lesional HS skin.

The PCoA plot (Fig.2) displays the significant differences between the three groups (overall and pairwise differences, Adonis PERMANOVA test, p < 0.001). There was no significant association between the number of species and the duration of the lesions or the diameter of the lesions (p>0.05).

In the three groups, no difference between the number of species was found (overall p = 0.66, lesional vs. non-lesional p = 0.97, lesional vs healthy controls p = 0.46, non-lesional vs. healthy p = 0.41, see Fig. 3a). However, as demonstrated in Fig. 3b, an increased Shannon diversity, due to increased taxonomic evenness, was found in group 2, compared to both group 1 (p = 0.018) and group 3 (p = 0.0025, overall p = 0.005), while group 1 and 3 were not significantly different (p = 0.22).

Differential abundance (DA) analyses at species and genus level yielded various differences between the groups (DA plots, Fig. 4a-4e). At genus level, the DA plot showed a significantly increased relative abundance of *Porphyromonas* and *Peptoniphilus spp* (microbiome type IV) in lesional skin samples versus healthy controls samples (Fig.4a).

Furthermore, *Propionibacterium sp.* showed a significantly higher relative abundance in healthy controls versus lesional skin (Fig.4a). At species level, a significantly higher relative abundance of *P. acnes* and *Corynebacterium mucifaciens* was found in the healthy control group compared to that of lesional HS skin (Fig.4b), indicating a possible beneficial (commensal) property of these species. This is also in congruence with the barplot (Fig.1) depicting the microbiome type V (*P. acnes*) as unique for healthy controls.

In lesional skin versus non-lesional skin, the relative abundance of *Porphyromonas and Peptoniphilus spp* remained significantly higher in lesional skin, both a species and genus level (Fig. 4c and Fig 4d). Conversely, a significantly reduced relative abundance of *Propionibacterium sp.* and P. acnes was found in lesional skin versus non-lesional (Fig. 4c and Fig 4d). DA analysis of non-lesional skin versus healthy controls only yielded four species that were significantly different (Fig. 4e).

In lesional skin, a significant association was found between microbiome types and location (p=0.037) with biopsies obtained from the groin showing a higher frequency of microbiome type IV. In non-lesional skin a significant association was also found between the microbiome types and location (p=0.038), with microbiome type II associated with biopsies taken from the axilla and again the inventors found microbiome type IV to be associated with biopsies obtained from the groin. In non-lesional skin, but not in lesional skin, gender had a significant association with microbiome (p=0.036), female patients were more likely to have microbiome type II.

Overall, the NGS approach carried out by the inventors provided a previously unreported characterization of the skin microbiome in HS. The data demonstrated that the microbiome in HS differs significantly from that of healthy controls, in both lesional and non-lesional HS skin (Figs. 1 and 2). In addition, the DA figures (Fig. 4a-e) showed significant differences at both genus level and for the species between the groups. Surprisingly, there were no differences in the overall number of the species found in each group (Fig.3a). However, in non-lesional skin, a significantly increased taxonomic evenness was found (Fig.3b), indicating a possible imbalance (dysbiosis) of the microbial community in clinically normal-appearing HS skin.

The presence of anaerobic bacteria such as *Porphyromonas sp.* (gram-negative rods) and *Peptoniphilus sp* (gram-positive cocci) in lesional HS (microbiome skin type IV) as well in non- lesional skin, have not previously been reported as important pathogenic bacteria in HS. Both bacteria belong to the spectrum of commensal bacteria on mucocutaneous surfaces and rarely colonize the skin.

Whereas it is known in the art that skin microbiome studies in intertriginous areas of healthy subjects have revealed *Corynebacterium sp.* and *Staphylococcus sp.* as abundant and important species, the present study showed that the microbiome skin type I (*Corynebacterium sp.*) appeared in all three groups, and may thus reflect the profiling of the indigenous intertriginous microbial community.

In non-lesional skin from HS patients, the inventors found a significantly increased diversity of the microbiota compared to HS lesional skin and healthy controls, while the overall number of species was the same, indicating that the non-lesional HS skin is characterized by a higher taxonomic evenness of several species.

In conclusion, the microbiome in non-lesional and lesional HS skin differ significantly from that of healthy controls.

### Example 2

### Application of saprophytic live non-pathogenic bacteria in Hidradenitis Suppurativa

In a recent Next Generation Sequencing (NGS) study, the inventors demonstrated Hidradenitis Suppurativa patients have a significantly different axillary microbiome compared to that of healthy controls. Thus, a proof of conduct study has now been conducted, exploring the clinical risk and efficacy of the application of a non-pathogenic microflora similar to a normal axillary microflora. In vivo application of a normal axillary microflora in HS patients may inform us of the immediate clinical response or problems.

### Participants

### Inclusion criteria:

HS subjects above 18 years old who have read and understood oral - as well as written information in Danish or English. The HS diagnosis has to be verified by an experienced dermatologist. All cases have a verified diagnosis of HS (ICD-10 code: L73.2). All patients have an intermittent activity of HS in the axillary. However, at the time of investigation, there must be no signs of clinical flare-ups of HS (e.g. no swelling, reddish discoloration, pus or scarring) in axilla. It is furthermore required that the healthy control has no previous record of dermatological diseases or other systemic disease, which potentially could affect the cutaneous microbiome. Moreover, the axillary region from then healthy controls has to be clinically unaffected (without clinical signs of inflammation, scarring or swelling).

### Exclusion criteria:

Pregnancy, breastfeeding and subjects whom are deprived of their liberty according to the law on detention and other coercion in psychiatry cannot participate in the research project. Subjects who have received antibiotics (systemic or topical therapy) one month prior time at investigation are also excluded.

### Methods

The mixture of saprophytic live non-pathogenic bacteria is cultured from the axilla of one healthy controls. The non-pathogenic microflora that is applied originates from the axilla of healthy control who does not suffer from any dermatological diseases. The microflora is created by swabbing the healthy controls in axilla and immediately after culturing the bacteria on standard agar plates. The bacterial composition is overall similar to the normal axillary microflora of healthy controls.. The swab is then cultured on routine blood agar plates and the subsequently formed bacterial colonies are diluted with sterile water. The bacteria suspension is distributed in 2 ml sterile Eppendorf tubes. The bacterial solutions are then slightly diluted with sterile water.

Overview of experimental set-up (only axillary regions are examined).
1. Initially a clinical photo of the area of interest is obtained.
2. To achieve insight on the microbiological landscape the axillary area of interest is swabbed using an e-swab. The sample is then immediately stored at minus 80 degrees. This applies for all bacteriological samples.
3. At the axillary site where the subject (patient) reports an intermittent HS activity, the bacterial suspension carefully is applied using a sterile e-swab.
4. In order to occlude the area, a Tegaderm patch® (Minnesota, USA) is applied on the axillary area.
5. The cunject (patient) is then advised not to remove the patch within 6-8 hours or showering.
6. The next day the same procedure is repeated. The subject (patient) is now advised not to shower within the next 24 hours.
7. The final clinical evaluation occurs 14 days after the second visit. A third e-swab - and a clinical photo of the axillary area is obtained. The subject (patient) is thoroughly interviewed about any HS flare-ups or potential side effects since the application of the second solution of bacteria.

### Experimental design

Three HS patients were enrolled in this explorative case study. All patients experienced an intermittent activity of HS in the axillary regions. However, at the time of investigation none of the patients experienced any HS activity at the investigated axillary site. Moreover, none of the patients had received any antibiotics within one month.

In the three HS patients, saprophytic live non-pathogenic bacteria was applied to the axillary regions. The bacteria was obtained from a healthy control who had no signs of any skin diseases. The bacteria were cultured on standard agar plates. The bacteria was slightly diluted in sterile water and distributed in sterile Eppendorf tubes. The samples were all stored at minus 80 degrees.

At day one, an e-swab was initially obtained from the area of interest and analyzed using NGS. The bacterial solution was then applied at the axilla using a sterile e-swab. The area was occluded using a Tegaderm patch® (Minnesota, USA). The patients were instructed not to bath and not to remove the patch for a minimum of 6 hours.

At day two, the same procedure was repeated. However, prior application of the second bacterial solution, an e-swab was obtained from the investigated area and examined using NGS.

At day 16, a third e-swab was obtained from the investigated area and analyzed using NGS. Furthermore, the axillary areas were thoroughly evaluated clinically. Moreover, the patients were questioned about the potential HS activity in the investigated regions during the last 14 days.

### Potential side effects

In this trial, a bacterial culture is applied to the armpit of a subject (HS patient), derived from the armpit of a healthy volunteer. Since this bacterial flora is non-pathogenic and the skin area to which the culture is applied is clinically intact, the likelihood of potential infection or other skin irritation is extremely small. However, when using the patch, applied after the bacterial culture is put on, a little irritation may occur as the patch potentially tear hair from the armpit and pinch a little as it sits in a skin fold (armpit).

### Results

None of the patients experienced any HS activity between the application of the first bacterial solution and to the third evaluation 16 days after. None of the patients reported any side effects related to the application of the bacteria. The tested subjects (HS patients) experienced no flares of their HS during the observation period and no indications of immediate problems with the in vivo application of bacteria similar to those found in the normal flora of healthy skin to HS patients.

### Organization Applicant

Street : Sygehusvej 10
City : Roskilde
State :
Country : Denmark
PostalCode :
PhoneNumber :
FaxNumber :
EmailAddress :
<110> OrganizationName : Sjællands Universitetshospital

### Application Project

<120> Title : Saprophytic live bacteria for use in treatment of Hidradenitis
   Suppurativa
<130> AppFileReference : 57612DK01
   <140> CurrentAppNumber :
   <141> CurrentFilingDate : _-_-_

### Sequence

<213> OrganismName : DNA/RNA
<400> PreSequenceString :
   gccagcagcc gcggtaattc
20
   <212> Type : DNA/RNA
   <211> Length : 20 SequenceName : SEQ ID NO: 1 SequenceDescription :

### Sequence

<213> OrganismName : DNA/RNA
<400> PreSequenceString :
   acattcttgg caaatgcttt cgcag
25
   <212> Type : DNA/RNA
   <211> Length : 25 SequenceName : SEQ ID NO: 2 SequenceDescription :

### Sequence

<213> OrganismName : DNA/RNA
<400> PreSequenceString :
   cagccgcggt aattccagct c
21
   <212> Type : DNA/RNA
   <211> Length : 21
   SequenceName : SEQ ID NO: 3
   SequenceDescription :

### Sequence

<213> OrganismName : DNA/RNA
<400> PreSequenceString :
   ggtggtgccc ttccgtcaat
20
   <212> Type : DNA/RNA
   <211> Length : 20
   SequenceName : SEQ ID NO: 4
   SequenceDescription :

### Sequence

<213> OrganismName : DNA/RNA
<400> PreSequenceString :
   tggagggcaa gtctggtgcc
20
   <212> Type : DNA/RNA
   <211> Length : 20
   SequenceName : SEQ ID NO: 5
   SequenceDescription :

### Sequence

<213> OrganismName : DNA/RNA
<400> PreSequenceString :
   acggtatctg atcgtcttcg atccc
25
   <212> Type : DNA/RNA
   <211> Length : 25
   SequenceName : SEQ ID NO: 6
   SequenceDescription :

### Sequence

<213> OrganismName : DNA/RNA
<400> PreSequenceString :
   actcctaygg grbgcascag
20
   <212> Type : DNA/RNA
   <211> Length : 20
   SequenceName : SEQ ID NO: 7
   SequenceDescription :

### Sequence

<213> OrganismName : DNA/RNA
<400> PreSequenceString :
   agcgtggact acnngggtat ctaat
25
   <212> Type : DNA/RNA
   <211> Length : 25
   SequenceName : SEQ ID NO: 8
   SequenceDescription :

## Claims

1. Saprophytic live bacteria mixture originating from the axillia of a human donor not suffering from Hidradenitis Suppurativa and who is having no previous record of dermatological diseases or other systemic diseases, which potentially could affect the cutaneous microbiome of the donor, for use in topical treatment of Hidradenitis Suppurativa in intertriginous skin areas of a human subject, wherein said saprophytic live bacteria mixture comprises at least the following: *Corynebacterium spp, Acinetobacter spp, Moraxella spp, Staphylococcus spp, Anaerococcus spp* and *Propionibacterium spp,* wherein the *Propionibacterium spp* of the mixture is dominated by *Propionibacterium acnes* and with the proviso that the mixture does not contain *Porphyromonas* and *Peptoniphilus spp*

2. Saprophytic live bacteria mixture for use according to claim 1, wherein the *Staphylococcus spp* are selected from the group consisting of *Staphylococcus epidermidis, Staphylococcus deformans, Staphylococcus caprae or Staphylococcus capitis.*

3. Saprophytic live bacteria mixture for use according to any of claims 1-2, wherein the intertriginous areas are areas of the axilla or the groin.

4. Saprophytic live bacteria mixture for use according to any of claims 1-3, wherein the concentrations of topically applied bacteria are between 1-10⁹ bacteria/mL culture medium.

5. Saprophytic live bacteria mixture for use according to any of claims 1-4, wherein the bacteria are topically applied directly to the skin via sprays, roll-on deodorants, creams, fats, pastes, foams, gels, lotions or ointments.

6. Saprophytic live bacteria mixture for use according to any of claims 1-5, wherein the period of treatment is between 2-180 days.

7. Saprophytic live bacteria mixture for use according to any one of claims 1-6, wherein the period between consecutive treatments is between 2-180 days.

8. Saprophytic live bacteria mixture for use according to any of claims 1-7, wherein the human subject is a female.

9. Saprophytic live bacteria mixture for use according to any of claims 1-7, wherein the human subject is a male.

10. Saprophytic live bacteria mixture for use according to any of claims 1-9, wherein the percentage ratios of bacterial CFU in the mixture are:
• 5-95 % CFU of *Corynebacterium spp:* 5-95 % CFU *Acinetobacter spp* and *Moraxella spp,*
• 5-95 % CFU of *Corynebacterium spp:* 5-95 % CFU of *Staphylococcus spp* and *Anaerococcus spp,*
• 5-95 % CFU of *Corynebacterium spp:* 5-95 % CFU of *Propionibacterium spp*
• 5-95 % CFU of *Acinetobacter spp* and *Moraxella spp:* 5-95 % CFU of *Staphylococcus spp* and *Anaerococcus spp*
• 5-95 % CFU of *Acinetobacter spp* and *Moraxella spp:* 5-95 % CFU of *Propionibacterium spp*
• 5-95 % CFU of *Staphylococcus spp* and *Anaerococcus spp:* 5-95 % CFU of *Propionibacterium spp.*

11. Method of diagnosing Hidradenitis Suppurativa (HS) in a human subject, wherein the method comprises at least the following consecutive steps:
(i) providing a biopsy sample that has been obtained by taking one or more skin biopsy sample(s) from intertriginous areas of said subject where at least one visible hair follicle is present,
(ii) extracting the DNA from the obtained biopsy sample(s),
(iii) amplifying and purifying the extracted DNA,
(iv) sequencing the amplified DNA by use of next-generation sequencing (NGS) targeting both 16S and 18S ribosomal RNA,
(v) based on the obtained NGS results, determine of the microbiome of bacteria contained in the biopsy sample(s), thereby
(vi) classifying the bacteria as *Corynebacterium spp, Acinetobacter spp* and *Moraxella spp, Staphylococcus spp* and *Anaerococcus spp, Porphyromonas* and *Peptoniphilus spp* and *Propionibacterium spp,*
thereby;
(1) if an absence of *Porphyromonas* and *Peptoniphilus spp* is observed this is an indication of skin not affected with HS, and
(2) if an absence of *Propionibacterium spp* is observed this is an indication of HS-affected skin, and
(3) if a predominance of *Porphyromonas* and *Peptoniphilus spp* is/are observed this is an indication of HS-affected skin.

## Patentansprüche

1. Gemisch von lebenden Saprophyten-Bakterisn, das aus der Achselhöhle eines menschlichen Spenders stammt, der nicht an Hidradenitis Suppurativa leidet und der keine Vorgeschichte an Hautkrankheiten oder anderen systemischen Krankheiten aufweist, die das kutane Mikrobiom des Spenders potentiell beeinträchtigen könnten, zur Verwendung bei der topischen Behandlung von Hidradenitis Suppurativa in intertriginösen Hautbereichen eines menschlichen Individuums, wobei das Gemisch von lebenden Saprophyten-Bakterisn zumindest Folgendes umfasst: *Corynebacterium spp., Acinetobacter spp., Moraxella spp., Staphylococcus spp., Anaerococcus spp.* und *Propionibacterium spp.,* wobei das *Propionibacterium spp.* des Gemischs durch *Propionibacterium acnes* beherrscht wird, und mit der Maßgabe, dass das Gemisch nicht *Porphyromonas* und *Peptoniphilus spp.* enthält.

2. Gemisch von lebenden Saprophyten-Bakterisn zur Verwendung nach Anspruch 1, wobei die *Staphylococcus spp.* aus der Gruppe bestehend aus *Staphylococcus epidermidis, Staphylococcus deformans, Staphylococcus caprae oder Staphylococcus capitis* ausgewählt sind.

3. Gemisch von lebenden Saprophyten-Bakterisn zur Verwendung nach einem der Ansprüche 1-2, wobei die intertriginösen Bereiche Bereiche der Achselhöhle oder der Leistenbeuge sind.

4. Gemisch von lebenden Saprophyten-Bakterisn zur Verwendung nach einem der Ansprüche 1-3, wobei die Konzentrationen von topisch aufgetragenen Bakterien bei 1-10⁹ Bakterien/ml Kulturmedium liegen.

5. Gemisch von lebenden Saprophyten-Bakterisn zur Verwendung nach einem der Ansprüche 1-4, wobei die Bakterien durch Sprays, Roll-on-Deodorants, Cremes, Fette, Pasten, Schäume, Gele, Lotionen oder Salben topisch direkt auf die Haut aufgetragen werden.

6. Gemisch von lebenden Saprophyten-Bakterisn zur Verwendung nach einem der Ansprüche 1-5, wobei der Behandlungszeitraum bei 2-180 Tagen liegt.

7. Gemisch von lebenden Saprophyten-Bakterisn zur Verwendung nach einem der Ansprüche 1-6, wobei der Zeitraum zwischen aufeinanderfolgenden Behandlungen bei 2-180 Tagen liegt.

8. Gemisch von lebenden Saprophyten-Bakterisn zur Verwendung nach einem der Ansprüche 1-7, wobei das menschliche Individuum eine Frau ist.

9. Gemisch von lebenden Saprophyten-Bakterisn zur Verwendung nach einem der Ansprüche 1-7, wobei das menschliche Individuum ein Mann ist.

10. Gemisch von lebenden Saprophyten-Bakterisn zur Verwendung nach einem der Ansprüche 1-9, wobei die prozentualen Anteile von bakteriellen KBE in dem Gemisch wie folgt sind:
• 5-95 % KBE von *Corynebacterium spp.:* 5-95 % KBE von *Acinetobacter spp.* und *Moraxella spp.,*
• 5-95 % KBE von *Corynebacterium spp.:* 5-95 % KBE von *Staphylococcus spp.* und *Anaerococcus spp.,*
• 5-95 % KBE von *Corynebacterium spp.:* 5-95 % KBE von *Propionibacterium spp.*
• 5-95 % KBE von *Acinetobacter spp.* und *Moraxella spp.:* 5-95 % KBE von *Staphylococcus spp.* und *Anaerococcus spp.*
• 5-95 % KBE von *Acinetobacter spp.* und *Moraxella spp.:* 5-95 % KBE von *Propionibacterium spp.*
• 5-95 % KBE von *Staphylococcus spp.* und *Anaerococcus spp.:* 5-95 % KBE von *Propionibacterium spp.*

11. Verfahren zum Diagnostizieren von Hidradenitis Suppurativa (HS) bei einem menschlichen Individuum, wobei das Verfahren zumindest die folgenden aufeinanderfolgenden Schritte umfasst:
(i) Bereitstellen einer Biopsieprobe, die durch Entnehmen einer oder mehrerer Hautbiopsieproben aus intertriginösen Bereichen des Individuums, an denen zumindest ein sichtbares Haarfollikel vorhanden ist, erhalten wird,
(ii) Extrahieren der DNA aus den erhaltenen Biopsieproben,
(iii) Verstärken und Reinigen der extrahierten DNA,
(iv) Sequenzieren der verstärkten DNA durch Verwendung von Sequenzierung der nächsten Generation (next-generation sequencing - NGS), die sowohl auf 16S als auch 18S ribosomale RNA abzielt,
(v) basierend auf den erhaltenen NGS-Ergebnissen, Bestimmen des Mikrobioms von Bakterien, die in der/den Biopsieprobe(n) enthalten sind, dadurch
(vi) Klassifizieren der Bakterien als *Corynebacterium spp., Acinetobacter spp.* und *Moraxella spp., Staphylococcus spp.* und *Anaerococcus spp., Porphyromonas* und *Peptoniphilus spp.* und *Propionibacterium spp.,*
wobei;
(1) wenn ein Nichtvorhandensein von *Porphyromonas* und *Peptoniphilus spp.* festgestellt wird, dies ein Anzeichen dafür ist, dass die Haut nicht durch HS betroffen ist, und
(2) wenn ein Nichtvorhandensein von *Propionibacterium spp.* festgestellt wird, dies ein Anzeichen für eine HS-betroffene Haut ist, und
(3) wenn ein Überwiegen von *Porphyromonas* und *Peptoniphilus spp.* festgestellt wird, dies ein Anzeichen für HS-betroffene Haut ist.

## Revendications

1. Mélange de bactéries vivantes saprophytes provenant de l'aisselle d'un donneur humain ne souffrant pas d'hidrosadénite suppurée et qui ne présente pas d'antécédents de maladies dermatologiques ou d'autres maladies systémiques, qui pourraient potentiellement affecter le microbiome cutané du donneur, pour utilisation dans le traitement topique de l'hidrosadénite suppurée dans les zones cutanées intertrigineuses d'un sujet humain, ledit mélange de bactéries vivantes saprophytes comprenant au moins les bactéries suivantes : *Corynebacterium spp, Acinetobacterspp, Moraxella spp, Staphylococcus spp, Anaerococcus spp* et *Propionibacterium spp,*
ledit *Propionibacterium spp* du mélange étant dominé par *Propionibacterium acnes* et à condition que le mélange ne contienne pas *de Porphyromonas* et *de Peptoniphilus spp.*

2. Mélange de bactéries vivantes saprophytes pour utilisation selon la revendication 1, lesdits *Staphylococcus spp* étant choisis dans le groupe constitué par *Staphylococcus epidermidis, Staphylococcus deformans, Staphylococcus caprae ou Staphylococcus capitis.*

3. Mélange de bactéries vivantes saprophytes pour utilisation selon l'une quelconque des revendications 1 à 2, lesdites zones intertrigineuses étant des zones de l'aisselle ou de l'aine.

4. Mélange de bactéries vivantes saprophytes pour utilisation selon l'une quelconque des revendications 1 à 3, les concentrations de bactéries appliquées localement étant comprises entre 1 à 10⁹ bactéries/ml de milieu de culture.

5. Mélange de bactéries vivantes saprophytes pour utilisation selon l'une quelconque des revendications 1 à 4, lesdites bactéries étant appliquées localement directement sur la peau via des sprays, des déodorants à bille, des crèmes, des graisses, des pâtes, des mousses, des gels, des lotions ou des onguents.

6. Mélange de bactéries vivantes saprophytes pour utilisation selon l'une quelconque des revendications 1 à 5, la période de traitement étant comprise entre 2 à 180 jours.

7. Mélange de bactéries vivantes saprophytes pour utilisation selon l'une quelconque des revendications 1 à 6, la période entre des traitements consécutifs étant comprise entre 2 à 180 jours.

8. Mélange de bactéries vivantes saprophytes pour utilisation selon l'une quelconque des revendications 1 à 7, ledit sujet humain étant une femme.

9. Mélange de bactéries vivantes saprophytes pour utilisation selon l'une quelconque des revendications 1 à 7, ledit sujet humain étant un homme.

10. Mélange de bactéries vivantes saprophytes pour utilisation selon l'une quelconque des revendications 1 à 9, les rapports en pourcentage de CFU bactérienne dans le mélange étant :
• 5 à 95 % de CFU de *Corynebacterium spp* : 5 à 95 % de CFU *d'Acinetobacter spp* et *de Moraxella spp,*
• 5 à 95 % de CFU de *Corynebacterium spp* : 5 à 95% de CFU de *Staphylococcus spp* et d'*Anaerococcus spp,*
• 5 à 95 % de CFU de *Corynebacterium spp* : 5 à 95 % de CFU de *Propionibacterium spp*
• 5 à 95 % de CFU d'*Acinetobacter spp* et de *Moraxella spp* : 5 à 95 % de CFU de *Staphylococcus spp* et d'*Anaerococcus spp*
• 5 à 95 % de CFU d'*Acinetobacter spp* et de *Moraxella spp* : 5 à 95 % de CFU de *Propionibacterium spp*
• 5 à 95 % de CFU de *Staphylococcus spp* et d'*Anaerococcus spp* : 5 à 95 % de CFU de *Propionibacterium spp.*

11. Procédé de diagnostic de l'hidrosadénite suppurée (HS) chez un sujet humain, ledit procédé comprenant au moins les étapes consécutives suivantes :
(i) la fourniture d'un échantillon de biopsie qui a été obtenu en prélevant un ou plusieurs échantillons de biopsie cutanée dans les zones intertrigineuses dudit sujet où au moins un follicule pileux visible est présent,
(ii) l'extraction de l'ADN du ou des échantillons de biopsie obtenus,
(iii) l'amplification et la purification de l'ADN extrait,
(iv) le séquençage de l'ADN amplifié à l'aide d'un séquençage nouvelle génération (NGS) ciblant à la fois l'ARN ribosomique 16S et 18S,
(v) sur la base des résultats obtenus par NGS, la détermination du microbiome des bactéries contenues dans le ou les échantillons de biopsie,
(vi) la classification des bactéries en *tant que Corynebacterium spp, Acinetobacter spp* et *Moraxella spp, Staphylococcus spp* et *Anaerococcus spp, Porphyromonas* et *Peptoniphilus spp* et *Propionibacterium spp,*
ainsi :
(1) si une absence de *Porphyromonas* et *de Peptoniphilus spp* est observée, cela indique que la peau n'est pas affectée par la HS, et
(2) si une absence de *Propionibacterium spp* est observée, cela indique une peau affectée par la HS, et
(3) si une prédominance de *Porphyromonas* et *de Peptoniphilus spp* est/sont observée(s), cela indique une peau affectée par la HS.
